# EUROPEAN PATENT SPECIFICATION

(11) **EP 2 005 883 B1**
(45) Date of publication and mention of the grant of the patent: **05.11.2014**
(21) Application number: 07739144.9
(22) Date of filing: 20.03.2007
(51) Int. Cl.: A61B 5/0255, A61B 5/021

(54) **ELECTRONIC SPHYGMOMANOMETER FOR PREVENTING ALTERATION OF MEASUREMENT VALUES**
ELEKTRONISCHES SPHYGMOMANOMETER ZUM VERHINDERN VON ÄNDERUNGEN VON MESSWERTEN
SPHYGMOMANOMÈTRE ÉLECTRONIQUE POUR PRÉVENIR LA MODIFICATION DES VALEURS MESURÉES

(30) Priority: 05.04.2006 US 397962
(43) Date of publication of application: 24.12.2008
(73) Proprietor: Omron Healthcare Co., Ltd., Kyoto 617-0002 (JP)
(72) Inventor: TAKEOKA, Kohhei, Kyoto 617-0002 (JP); LI, Jim Jun, Bannockburn, Illinois 60015 (US); YAMASHITA, Shingo, Kyoto 617-0002 (JP)
(74) Representative: Kilian Kilian & Partner
(86) International application number: PCT/JP2007/055701
(87) International publication number: WO 2007/114050

(56) References cited:
- JP-A- 5 335 992
- JP-A- 05 203 161
- JP-A- 05 334 136
- JP-A- 05 335 992
- JP-A- 2005 224 440
- JP-A- 2005 224 440
- JP-U- 3 020 497
- US-B1- 6 449 583
- US-B1- 6 506 162

## Description

### TECHNICAL FIELD

The present invention relates to electronic sphygmomanometers, in particular, to an electronic sphygmomanometer having a function of erasing measurement values and/or changing set values in addition to the function of measuring blood pressure.

### BACKGROUND ART

A home electronic sphygmomanometer generally has a function of storing measurement data of the blood pressure, and displaying the blood pressure value measured in the past. In such sphygmomanometer, there are times one would want to erase the past measurement data, for example, when handed over by another person. Thus, an electronic sphygmomanometer having a function of erasing the past measurement data exists from the prior art.

Japanese Laid-Open Patent Publication No. 2005-224440 (hereinafter referred to as patent document 1) discloses a sphygmomanometer capable of erasing only the measurement results the user wants to erase out of a plurality of measurement results measured by the user and stored in a memory. The user can then erase only the measurement data of another person.

A sphygmomanometer having a function of storing the blood pressure data in correspondence to a measurement condition such as date and time, and displaying the past blood pressure values in association with the condition in time of measurement is also known. Such sphygmomanometer also has a function of setting or changing the value that becomes a reference of the measurement condition before the blood pressure measurement.

Japanese Laid-Open Patent Publication No. 2002-272686 (hereinafter referred to as patent document 2) and Japanese Laid-Open Patent Publication No. 2002-272687 (hereinafter referred to as patent document 3) describe a sphygmomanometer including a time setting switch for setting/correcting time, and a time forward switch for fast-forward or fast-rewind when setting/correcting the time. Changing the date/time by operating such switches is disclosed. The user can correct the date/time when the date/time in the sphygmomanometer are wrong. Therefore, the blood pressure value is prevented from being stored with the wrong date and time (measurement condition).
[Patent document 1] Japanese Laid-Open Patent Publication No. 2005-224440
[Patent document 2] Japanese Laid-Open Patent Publication No. 2002-272686
[Patent document 3] Japanese Laid-Open Patent Publication No. 2002-272687

An electronic sphygmomanometer according to the preamble of claim 1 is known from US-31-6 506 162.

### DISCLOSURE OF THE INVENTION

### PROBLEMS TO BE SOLVED BY THE INVENTION

However, inconveniences may arise to the manager (doctors etc.) of the subject if the user can freely erase the measurement data or change the set value. For instance, suppose the manager instructs the subject to measure the blood pressure every day. Normally, the manager grasps change in blood pressure of the subject and the like by browsing at the measurement results on a later date. However, if the measurement data can be freely erased, the subject might erase the data if the subject is not satisfied with the measurement result, which leads to alteration of measurement data.

Even if the manager specifies to the subject a period of time for blood pressure measurement, the subject can alter the measurement time by changing the time setting.

In view of solving the above problems, the present invention aims to provide an electronic sphygmomanometer capable of disabling erasing of measurement values and/or changing of set values by the subject.

### MEANS FOR SOLVING THE PROBLEMS

An electronic sphygmomanometer according to the present invention is as defined in claim 1.

Preferably, measurement result information further includes condition specifying data for specifying a measurement condition in blood pressure measurement in association with each blood pressure data; and the information changing function includes at least one of an erasing function of erasing the measurement result information stored in the first storage part or a changing function of changing a value that becomes a reference of the measurement condition.

The setting portion preferably sets the activation of the second function group to enable when determined as started with the specific operation by the determining portion, and sets the activation of the second function group to disable when determined as started with an operation different from the specific operation by the determining portion.

The electronic sphygmomanometer preferably further includes a timer for measuring time; wherein the condition specifying data includes timing data from the timer in the blood pressure measurement.

A second storage part for storing history information of the start-up with the specific operation is preferably arranged; wherein the second function group further includes a second displaying function of displaying the history information stored in the second storage part on the display unit.

The electronic sphygmomanometer preferably further includes a timer for measuring date and time, wherein the activation controlling part further includes a data storing portion for storing start-up date and time data in the second storage part as the history information based on an output from the timer when determined as started with the specific operation by the determining portion.

Alternatively, the activation controlling part preferably further includes a counting portion for counting the number of start-ups with the specific operation when determined as started with the specific operation by the determining portion, and a data storing portion for storing data of the counted number of start-ups in the second storage part as the history information.

The specific operation is preferably an operation different from an explicit normal start-up operation.

The first input unit preferably includes a plurality of switches that can be pushed by the user; and the determining portion determines as started with the specific operation when two or more predetermined switches of the plurality of switches are simultaneously pushed by the user.

Alternatively, the electronic sphygmomanometer may preferably further include a second input unit arranged hidden from the outside; wherein the determining portion determines as started with the specific operation when the second input unit is operated.

The invention, together with other objects, features, aspects, and advantages thereof, may best be understood by reference to the following description of the presently preferred embodiment together with the accompanying drawings.

### BRIEF DESCRIPTION OF THE DRAWINGS

Fig. 1 is a view showing an appearance of an electronic sphygmomanometer according to an embodiment and its variant of the present invention.
Fig. 2 is a block diagram showing a hardware configuration of the electronic sphygmomanometer according to the embodiment and its variant of the present invention.
Fig. 3A is a view showing a configuration example of a memory according to the embodiment of the present invention.
Fig. 3B is a function block diagram showing functions of a CPU (Central Processing Unit) in the electronic sphygmomanometer of the embodiment of the present invention.
Fig. 4 is a flowchart showing a measurement related process in the embodiment of the present invention.
Fig. 5 is a view showing one example of a screen displayed when the menu switch is pushed in the embodiment of the present invention.
Fig. 6A is a view showing a configuration example of a memory according to a variant of the embodiment of the present invention.
Fig. 6B is a function block diagram of a CPU in an electronic sphygmomanometer of the variant of the embodiment of the present invention.
Fig. 7 is a flowchart showing the measurement related process according to the variant of the embodiment of the present invention.
Fig. 8 is a view showing one example of a screen displayed when the menu switch is pushed in the variant of the embodiment of the present invention.

### DESCRIPTION OF SYMBOLS

- 1: sphygmomanometer body
- 2: cuff
- 3: air tube
- 4: display unit
- 12, 12A: memory
- 13: timer
- 14: pressure sensor
- 15: oscillation circuit
- 16: pump
- 17: pump drive circuit
- 18: valve
- 19: valve drive circuit
- 20, 20A: CPU
- 21: operation unit
- 21.0: power switch
- 21.1: menu switch
- 21.2: set switch
- 21.3: measurement switch
- 21.4: left switch
- 21.5: right switch
- 21.6: memory switch
- 24: power supply unit
- 25: air bladder
- 30: blood pressure measuring unit
- 100: electronic sphygmomanometer
- 121: measurement result storage region
- 122: history storage region
- 201,201A: activation controlling part
- 203: measurement processing part
- 204: measurement data displaying part
- 205: measurement data erasing part
- 206: clock changing part
- 207: history displaying part
- 2011: determining portion
- 2012: setting portion
- 2013: history storing portion

### BEST MODE FOR CARRYING OUT THE INVENTION

### (Regarding configuration)

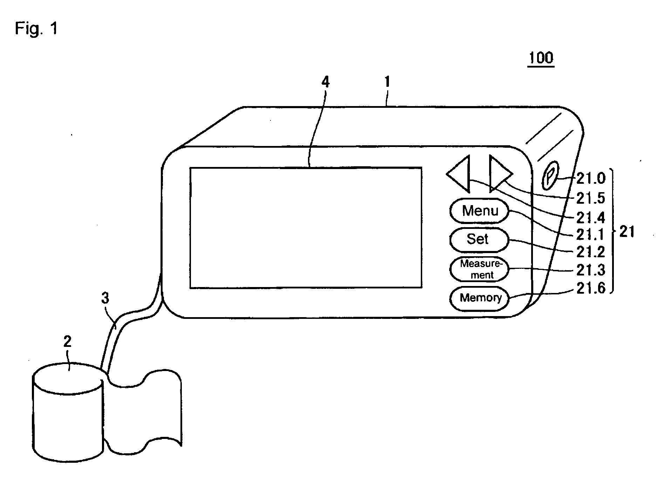

Fig. 1 is a view showing an appearance of an electronic sphygmomanometer 100 according to an embodiment of the present invention.

With reference to Fig. 1, the electronic sphygmomanometer 100 according to the present embodiment includes a sphygmomanometer body 1, a cuff 2 to be attached to a predetermined body site of a user to pressurize with air pressure, and an air tube 3 for connecting the sphygmomanometer body 1 and the cuff 2.

The sphygmomanometer body 1 includes a display unit 4 arranged to allow the user to check the displayed content, and an operation unit 21 for accepting input of instructions from the user. The display unit 4 is configured by an LCD (Liquid Crystal Display), and the like. The operation unit 21 includes a plurality of switches such as power switch 21.0, menu switch 21.1, set switch 21.2, measurement switch 21.3, left switch 21.4, right switch 21.5, and memory switch 21.6. The power switch 21.0 is a switch for inputting an instruction to turn ON/OFF the power. The menu switch 21.1 is a switch for inputting an instruction to menu display information on the functions of the electronic sphygmomanometer 100. The set switch 21.2 is a switch for executing a setting operation related to the information displayed on the display unit 4. The measurement switch 21.3 is a switch for starting the measuring operation of the blood pressure. The left switch 21.4 and the right switch 21.5 are switches for moving, for example, a cursor (not shown) displayed on the display unit 4 to the left (or upward) and the right (or downward), respectively. The memory switch 21.6 is a switch for inputting an instruction to display past measurement result information (hereinafter described). The operation unit 21 can accept input of instructions to activate one of a plurality of functions included in a first function group and a second function group of the electronic sphygmomanometer 100 by the user. The first function group and the second function group will be hereinafter described.

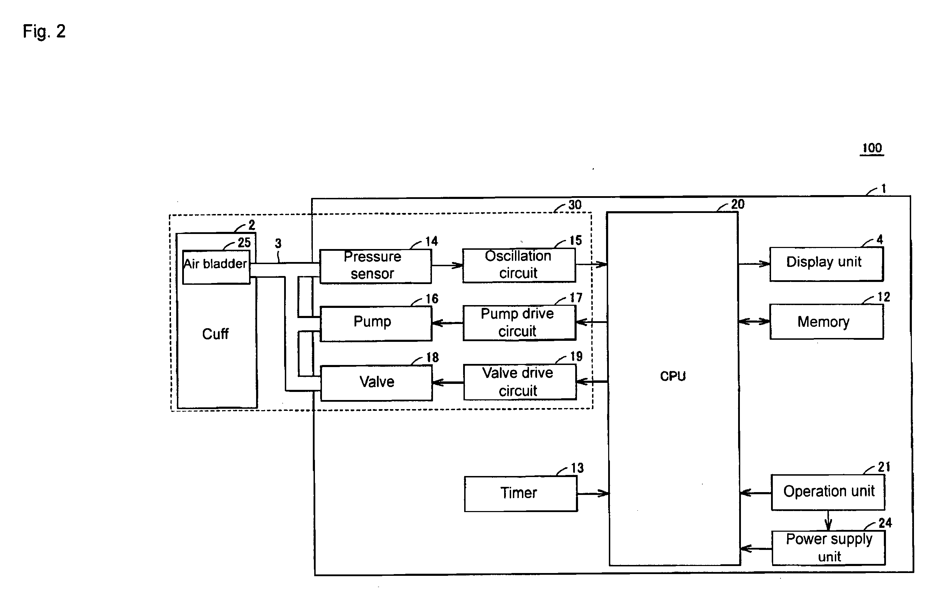

Fig. 2 is a block diagram showing a hardware configuration of an electronic sphygmomanometer 100 according to an embodiment of the present invention. With reference to Fig. 2, the electronic sphygmomanometer 100 includes a blood measuring unit 30 for measuring blood pressure, a CPU (Central Processing Unit) 20 for controlling and monitoring each unit in a concentrated manner, the display unit 4, a memory 12 for storing various data and programs, the operation unit 21, a timer 13 for performing a timing operation and outputting timing data, and a power supply unit 24. In the present embodiment, the blood pressure measuring unit 30 includes the cuff 2 described above, an air bladder 25 embedded in the cuff 2, a pressure sensor 14 which capacity changes by the pressure (hereinafter referred to as "cuff pressure") in the air bladder 25, an oscillation circuit 15 for outputting a signal of an oscillating frequency corresponding to a capacitance value of the pressure sensor 14 to the CPU 20, a pump 16 and a valve 18 for adjusting the pressure level of the cuff pressure, a pump drive circuit 17 for driving the pump 16, and a valve drive circuit 19 for adjusting the open/close degree of the valve 18. The air bladder 25 and the pressure sensor 14, the pump 16, and the valve 18 are connected by way of the air tube 3. The blood pressure measuring unit 30 is not limited to such configuration, and information (pulse wave information) for measuring the blood pressure merely needs to be detectable. The electronic sphygmomanometer 100 may also include a buzzer (not shown) for emitting an alarm sound.

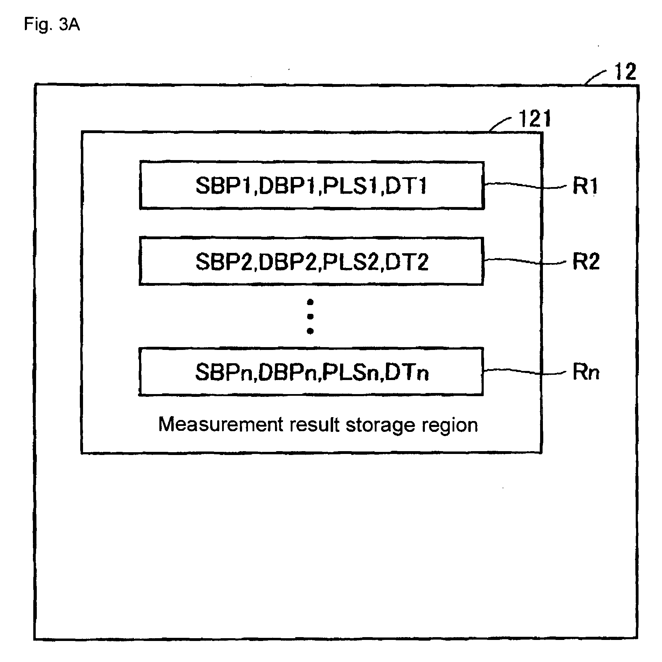

A configuration example of the memory 12 according to the present embodiment is shown in Fig. 3A. The memory 12 includes a measurement result storage region 121 for storing measurement result information.

With reference to Fig. 3A, measured blood pressure data and condition specifying data for specifying the measurement condition in the blood pressure measurement are stored in pairs in the measurement result storage region 121. In Fig. 3A, a record R in which the blood pressure value and the pulse rate, and the measurement date and time (e.g., date and time of start and end of measurement) are corresponded is stored for every blood pressure measurement. The systolic blood pressure SBP, the diastolic blood pressure DBP, the pulse rate data PLS, as well as the measurement date and time DT are stored in the record R. Such data merely need to be stored in each region in correspondence to every measurement, and the storage format is not limited to using the record R.

The electronic sphygmomanometer 100 according to the embodiment of the present invention has two types of start-up methods. One method is a start-up method with a normal operation (explicit start-up operation). The normal operation is the pushing (operating) of the power switch 21.0 in the present embodiment. A display such as "power ON/OFF" is preferably made on the surface of the power switch 21.0. The other method is a start-up method with a specific operation that is not explicit (operation different from the normal start-up operation). The specific operation is, for example, simultaneous pushing (operating) of the power switch 21.0, the set switch 21.2, and the measurement switch 21.3 in the present embodiment.

In the present embodiment, the electronic sphygmomanometer 100 operates in a lock mode (hereinafter described) if started with the normal operation. On the other hand, the electronic sphygmomanometer 100 operates in an unlock mode (hereinafter described) if started with the implicit specific operation. Thus, the start-up with the specific operation is also referred to as "unlock start-up" in the present embodiment.

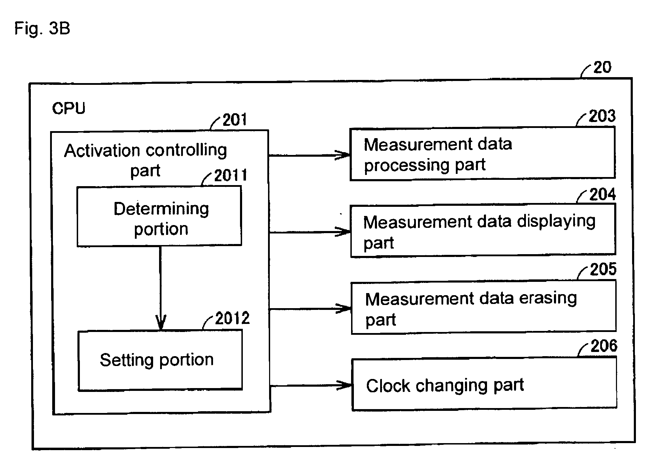

Fig. 3B is a function block diagram showing functions of the CPU 20 in the electronic sphygmomanometer 100 of the embodiment of the present invention. The CPU 20 includes an activation controlling part 201 for performing a control to activate one of a plurality of functions of the electronic sphygmomanometer 100 based on an instruction signal from the operation unit 21. The activation controlling part 201 includes a determining portion 2011 for determining whether started with the specific operation or not, and a setting portion 2012 for setting the necessity of activating the second function group based on such determination result. Specifically, the setting portion 2012 sets one of the modes of the lock mode or the unlock mode. The activation controlling part 201 activates the function corresponding to the instruction if the instruction from the user is the instruction to activate the function included in the first function group, or the instruction from the user is the instruction to activate the function included in the second function group, and is set as activatable by the setting portion 2012.

The first function group and the second function group will be described. The first function group has at least a blood pressure displaying function by the blood pressure measuring unit 30, and has a measurement data displaying function in addition to the displaying function in the present embodiment. Thus, the first function group can be defined as a basic function group of the electronic sphygmomanometer 100. The second function group, on the other hand, can be defined as an associated function group of the electronic sphygmomanometer 100. The second function group includes at least information changing function related to erasing the measurement value and/or changing the set value. The information changing function specifically includes at least one of an erasing function of erasing at least one of a plurality of measurement data (data of pair of measurement value and measurement condition) stored in the measurement result storage region 121, and a changing function of changing a value (set value) that becomes a reference of measurement condition before the blood pressure measurement. In the present embodiment, the information changing function includes both the erasing function and the changing function.

With reference again to Fig. 3B, the CPU 20 includes a measurement processing part 203, a measurement data displaying part 204, a measurement data erasing part 205, and a clock changing part 206 for performing processes respectively corresponding to the measuring function, the measurement data displaying function, the erasing function, and the changing function. The specific processes performed by each part will be briefly described below. Such processes may be realized through a known method.

The measurement processing part 203 controls the drive of the pump drive circuit 17 and the valve drive circuit 19, converts the signal from the oscillation circuit 15 to a pressure signal, and detects pressure. A predetermined algorithm is applied on the detected pressure data, and blood pressure values, that is, systolic blood pressure/diastolic blood pressure are calculated, and the pulse rate is calculated. The well-known procedures from the prior art can be applied to such measurement procedures, and thus the detailed description thereof will be omitted herein. The measurement processing part 203 acquires date and time of measurement based on the date and time data from the timer 13. The calculated blood pressure values and the pulse rate are displayed on the display unit 4, and the calculated blood pressure values and the pulse rate are stored in the measurement result storage region 121 in correspondence with the date and time data as the record R.

The measurement data displaying part 204 performs a process of reading out measurement result information stored in the measurement result storage region 121, and displaying the same on the display unit 4. More specifically, when the memory switch 21.6 is first pushed, the closest measurement data is read out and displayed on the display unit 4, and the past measurement data is read out and displayed one by one every time the pushing of the memory switch 21.6 is detected.

The measurement data erasing part 205 displays the measurement data one by one on the display unit 4, and erases the measurement data being displayed in units of records R by a predetermined operation (e.g., simultaneously operate the memory switch 21.6 and the right switch 21.5). The measurement data erasing part 205 may erase all the measurement data.

The clock changing part 206 performs a process of setting the time in the timer 13, and a process of changing the time setting (set value) in the timer 13.

According to the configuration described above, the activation controlling part 201 can activate the function corresponding to the instruction regardless of whether the lock mode or the unlock mode is set if the instruction from the user is an instruction to activate the function included in the basic function group. On the other hand, the activation controlling part 201 can activate the function corresponding to the instruction only when set to the unlock mode by the setting portion 2012 if the instruction from the user is an instruction to activate the function included in the associated function group.

The operation of each function block may be realized by executing software stored in the memory 12, and at least one of which may be realized by hardware.

In the present embodiment, the value (set value) that becomes a reference of the measurement condition is the time (date and time) in the electronic sphygmomanometer 100, but is not limited thereto. The value (set value) that becomes the reference of the measurement condition may be, in place of/in addition to date and time, number of times the electronic sphygmomanometer 100 successively executes the blood pressure measurement process (number of successive measurements), interval time of when the blood pressure measurement process is successively executed, a time (alarm time) for emitting an alarm sound to request for the start of measurement, and the like. That is, a process of changing the number of successive measurements, a process of changing the interval time, and/or a process of changing the alarm time may be carried out by the CPU 20 as a process corresponding to the changing function. If the measurement condition includes items such as number of successive measurements, interval time, and alarm time in time of measurement, each set value may be stored in a predetermined region of the memory 12. When the blood pressure is measured, each set value may be stored in the measurement result storage region 121 of the memory 12 in correspondence to the blood pressure value. Alternatively, when the set value corresponding to one of the items of the measurement condition is changed, the content of the changed item (e.g., item name and value after change) may be stored in a region (not shown) different from the measurement result storage region 121 of the memory 12 along with the date and time the set value is changed.

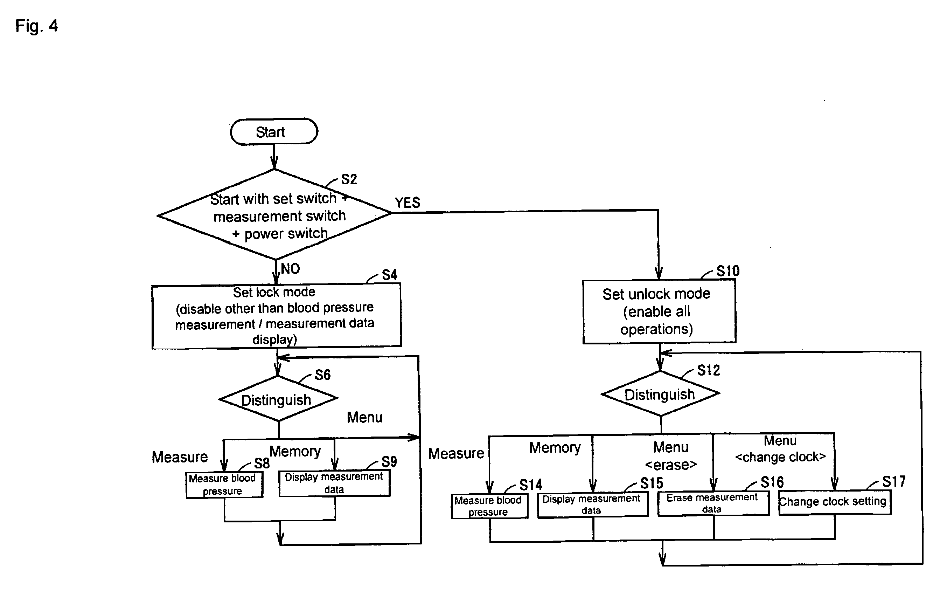

Fig. 4 is a flowchart showing a measurement related process according to the embodiment of the present invention. The process shown in the flowchart of Fig. 4 is stored in the memory 12 in advance as a program, and is realized by having the CPU 20 read out and execute the program. "Measurement related process" is the process related to blood pressure measurement, where the measurement related process includes the process by the measurement processing part 203, the process by the measurement data displaying part 204, the process by the measurement data erasing part 205, and the process by the clock changing part 206 in the present embodiment.

The process shown in Fig. 4 is a process that starts when the user operates at least the power switch 21.0 and the power is supplied to the CPU 20 through the power supply unit 24, and is a process executed when the power is turned ON. A power supply management program for managing the power ON state is also stored in the memory 12. The power supply management program determines whether started with the specific operation when the power is turned ON, and assumes that a flag indicating the determination result is stored in an internal memory (not shown).

With reference to Fig. 4, the determining portion 2011 determines whether started by simultaneously pushing the set switch 21.2, the measurement switch 21.3, and the power switch 21.0, that is, whether or not the start-up is executed with the special operation (step S2). Specifically, whether or not the start-up is executed with the specific operation is determined based on the state of the flag in the internal memory. If determined as started with the normal operation by the determining portion 2011 (NO in step S2), the process proceeds to step S4. If determined as the start-up with the specific operation by the determining portion 2011 (YES in step S2), the process proceeds to step S10.

In step S4, the setting portion 2012 sets the lock mode. That is, the activation of the associated function group other than the basic function group (measuring function and measurement data displaying function) is set to disable.

The activation controlling part 201 distinguishes the instruction from the user (step S6). More specifically, which switch of the operation unit 21 is operated is distinguished. If distinguished that the measurement switch 21.3 is pushed in step S6, the measurement processing part 203 executes the blood pressure measurement process (step S8). The process returns to step S6 after the process of step S8 is terminated.

If distinguished that the memory switch 21.6 is pushed in step S6, the measurement data displaying part 204 performs the process of displaying the measurement result information on the display unit 4 (step S9). The process returns to step S6 after the process of step S9 is terminated.

If distinguished that the menu switch 21.1 is pushed in step S6, the activation controlling part 201 waits.

Thus, in the lock mode, the operation of the switch for activating the functions included in the basic function group is valid, but the operation of the switch (menu switch 21.1) for activating the functions included in the associated function group is invalid.

In step S10, the setting portion 2012 sets the unlock mode. That is, the activation of the associated function group is set to enable (all operations are possible).

The activation controlling part 201 then distinguishes the instruction from the user (step S12). More specifically, which switch of the operation unit 21 is operated is distinguished, similar to step S6. If distinguished that the measurement switch 21.3 is pushed in step S12, the measurement processing part 203 executes the blood pressure measurement process (step S14). The process returns to step S12 after the process of step S14 is terminated.

If distinguished that the memory switch 21.6 is pushed in step S12, the measurement data displaying part 204 performs the process of displaying the measurement result information on the display unit 4 (step S15). The step returns to step S12 after the process of step S15 is terminated.

If distinguished that the menu switch 21.1 is pushed and "erase measurement data" is selected in step S12, the measurement data erasing part 205 performs the process of erasing the measurement result information (step S16). The process returns to step S12 after the process of step S16 is terminated.

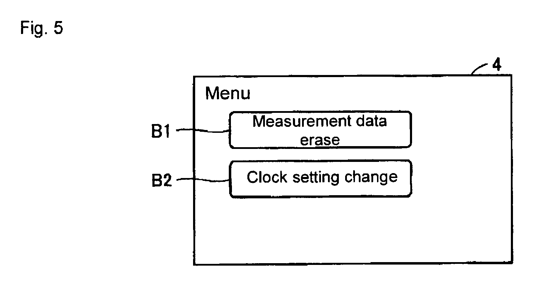

An example of a screen displayed when the menu switch 21.1 is pushed is shown in Fig. 5. With reference to Fig. 5, a measurement data erase button item B1 of and a clock setting change button item B2 are displayed on the display unit 4. The measurement data erase button item B1 is the item for inputting the instruction to erase the measurement data (instruction to activate the erasing function), and the clock setting change button item B2 is the item for inputting the instruction to change the clock (instruction to activate the changing function). The user pushes the left switch 21.4 and the right switch 21.5 to select either one of the items, and then pushes the set switch 21.2 to instruct activation of the function corresponding to the selected item.

If distinguished that the menu switch 21.1 is pushed and "change clock setting" is selected in step S12, the clock changing part 206 performs the process of changing the clock setting of the timer 13 (step S17). The process returns to step S12 after the process of step S17 is terminated.

Thus, the operation of the switches for activating all the functions is valid in the unlock mode.

As described above, the associated function group is activated only when started with the special operation, and thus alternation of the measurement values and the measurement conditions by the subject can be prevented.

### (Variant)

According to the embodiment described above, the subject who does not know the specific operation cannot erase the measurement values nor change the set values. However, there is a possibility the subject will find out about the specific operation in some way. The subject might alter the measurement values and the set values in this case. Thus, the history of start-up with the specific operation can be stored, and the possibility of alternation can be notified to the manager. Such operation of the electronic sphygmomanometer will be described below as a variant.

The outer appearance and the hardware configuration of an electronic sphygmomanometer according to a variant are similar to the electronic sphygmomanometer 100 of the above-described embodiment, and thus the reference numerals shown in Fig. 1 and Fig. 2 are used here as well. The memory 12 and the CPU 20 will be referred to as memory 12A and CPU 20A, respectively, in the variant due to the difference in the functions (controls).

The difference with the above-described embodiment will be described.

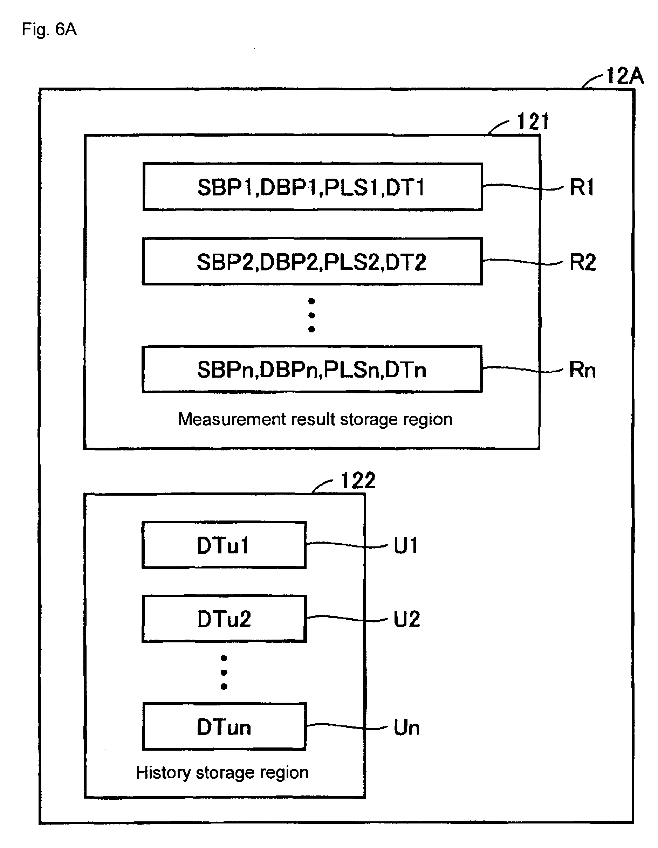

Fig. 6A is a view showing a configuration example of the memory 12A according to the variant of the present embodiment. In the variant, the memory 12A further includes a history storage region 122 for storing history information of the start-up with the specific operation, in addition to the measurement result storage region 121 described above.

With reference to Fig. 6A, information for specifying that start-up is executed with the specific operation, for example, start-up date and time data DTu is stored in the history storage region 122. The history storage region can store a predetermined number of (e.g., five) start-up date and time data DTu, and the most recent data is preferably overwritten on the oldest data.

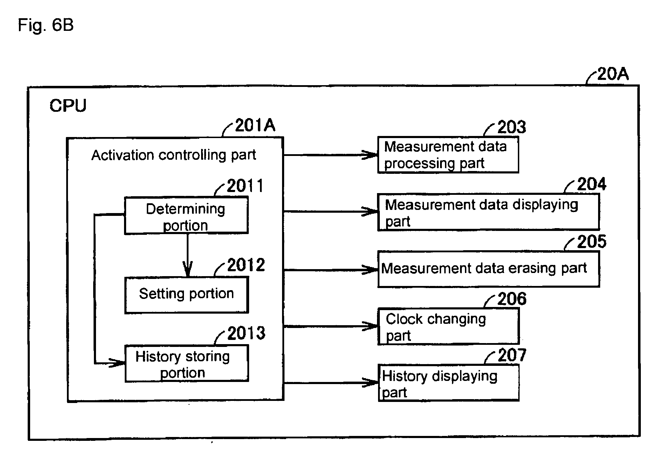

Fig. 6B is a function flock diagram of the CPU 20A in the electronic sphygmomanometer 100 according to the variant of the embodiment of the present invention. With reference to Fig. 6B, the activation controlling part 201 A further includes a history storing portion 2013 in addition to the determining portion 2011 and the setting portion 2012, compared to the above-described embodiment. The CPU 20A also includes a history displaying part 207 in addition to the activation controlling part 201, the measurement processing part 203, the measurement data displaying part 204, the measurement data erasing part 205, and the clock changing part 206.

The history storing portion 2013 stores the data of date and time in time of unlock start-up in the history storage region 122 as the start-up date and time data DTu.

The history displaying part 207 performs the process of reading out the start-up date and time data DTu stored in the history storage region 122, and displaying the same on the display unit 4.

In the variant, the history displaying function is included in the associated function group. The relevant function may be included in the basic function group as it differs from the information changing function.

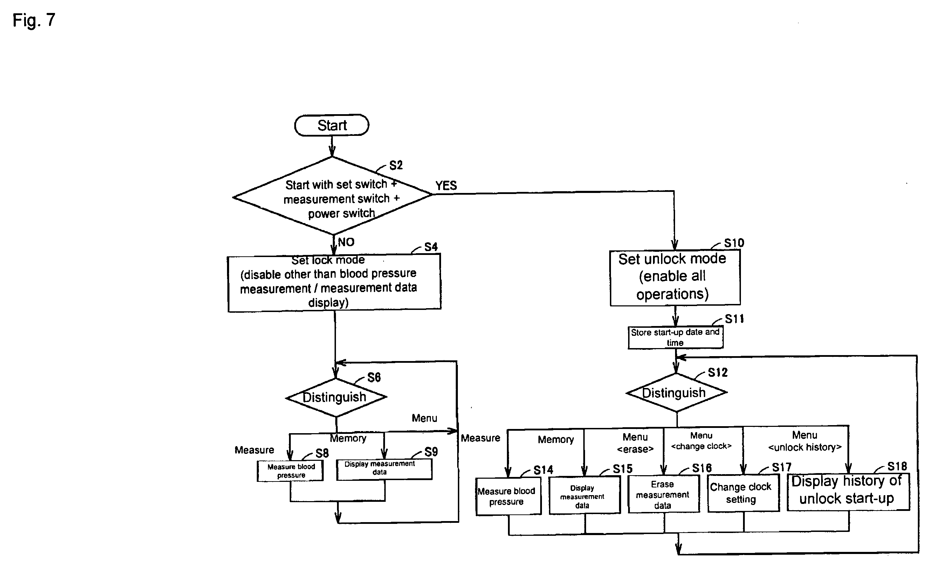

Fig. 7 is a flowchart showing the measurement related process according to the variant of the embodiment of the present invention. The process shown in the flowchart of Fig. 7 is also stored in the memory 12A in advance as a program, and is realized by having the CPU 20A read out and execute the program. In the variant, the measurement related process includes processes by the history displaying part 207. Same reference numerals are denoted for the processes similar to the flowchart of Fig. 4, and the description thereof will not be repeated.

With reference to Fig. 7, a process of storing start-up date and time (step S11) is inserted between step S10 and step S12. Specifically, in step S11, the history storing portion 2013 stores the data on the date and time of start-up with the specific operation in the history storage region 122 based on the timing data from the timer 13. The processes of step S10 and step S11 may be reversed.

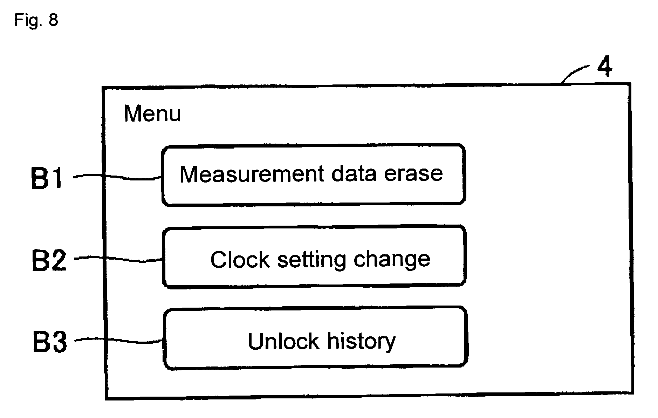

One example of a screen displayed when the menu switch 21.1 is pushed in step S12 in the variant is shown in Fig. 8. With reference to Fig. 8, an unlock history button item B3 is displayed in addition to the measurement data erase button item B1 and the clock setting change button item B2 on the display unit 4. The unlock history button item B3 is the item for inputting the instruction to display the history of the unlock start-up (instruction to activate the history displaying function).

. With reference again to Fig. 7, if distinguished that the menu switch 21.1 is pushed and the unlock history button item B3 is selected in step S12, the history displaying part 207 performs the process of displaying the history (date and time data DTu) of the unlock start-up on the display unit 4 (step S18). The process returns to step S12 after the process of step S18 is terminated.

Therefore, in the variant, the history information of the unlock start-up can be checked, and the manager can recognize the possibility of data alteration by the subject. Furthermore, since the function of erasing the history information is not included in the basic function group nor in the associated function group, whether or not the subject has unlock started up can be reliably stored.

Here, the history information is the information of date and time in time of unlock start-up, but is not limited thereto. The history information may be information on the number of unlock start-up. In this case, the history storing portion 2013 counts the number of unlock start-ups within a predetermined period (e.g., one week), and stores the count value in the history storage region 122.

Alternatively, the history information may include both the information on date and time and the information on number of unlock start-ups. In this case, the start-up date and time data and the count value data may both be stored in the history storage region 122.

As described above, the associated function group is selected by pushing the menu switch 21.1 in the present embodiment and its variant. However, the switches assigned to the respective functions (erasing function, changing function, history displaying function) included in the associated function group may also be arranged in the operation unit 21. In this case, the menu switch 21.1 does not need to be arranged in the operation unit 21.

Alternatively, the basic function group and the associated function group both may be selectable by a common switch (e.g., menu switch 21.1). In this case, the measurement switch 21.3 and the memory switch 21.6 become unnecessary. In the unlock mode, if the menu switch 21.1 is pushed, the activation controlling part 201, 201 A displays, for example, a plurality of items (first button) for having all functions included in the basic function group to be selectable, and an item (second button) for further displaying the details of the associated function group on the display unit 4. In the lock mode, on the other hand, if the menu switch 21.1 is pushed, the activation controlling part 201, 201 A displays, for example, the plurality of first buttons, and displays in gray the second button on the display unit 4. Thus, the functions included in the associated function group may not be displayed in the lock mode.

In the embodiment and the variant, the specific operation for the unlock start-up corresponds to simultaneously pushing a predetermined plurality of switches (include power switch 21.0) included in the operation unit 21. However, the specific operation is not limited to such mode. For instance, a switch dedicated for unlock start-up (hereinafter referred to as "hidden switch") (not shown) may be arranged at a position hidden from the outside (position difficult to be recognized by the user) such as the bottom surface (surface opposite to the surface arranged with the display unit 4) of the sphygmomanometer body 1. That is, the hidden switch is the switch for inputting the instruction to set the associated function group to be activatable. In this case, the specific operation may be to simultaneously push the power switch 21.0 and the hidden switch (not shown), or the specific operation may be to push only the hidden switch (not shown).

Although the invention has been described in detail above, the description is merely illustrative and is not restrictive, and it should be apparently recognized that the scope of the invention is limited only by the appended Claims.

## Claims

1. An electronic sphygmomanometer comprising:
a blood pressure measuring unit (30) adapted to measure blood pressure;
a first storage part (121) adapted to store measurement result information including past measured data of the blood pressure measured by the blood pressure measuring unit; and
a first input unit (21) adapted to enable a user to input an instruction to activate one of a plurality of functions included in a basic function group and an associated function group of the electronic sphygmomanometer, the basic function group including a measuring function by the blood pressure measuring unit, and the associated function group including an information changing function related to erasing a measurement value or changing a set value; and
an activation controlling part (201) adapted to perform a control to activate one of the plurality of functions based on the instruction from the first input unit; **characterized in that**
the activation controlling part includes
a determining portion (2011) adapted to determine whether the electronic sphygmomanometer is started-up with a normal operation or with a specific operation,
a setting portion (2012) adapted to set, when it is determined by the determining portion (2011) that the electronic sphygmomanometer is started-up with the specific operation, an unlock mode enabling activation of a function included in the associated function group and to set, when it is determined by the determining portion (2011) that the electronic sphygmomanometer is started-up with the normal operation, a lock mode disabling the activation of the associated function group other than the basic function group;
the activation controlling part (201) being further adapted to activate the function corresponding to the instructionregardless of whether the lock mode or unlock mode is set if the instruction from the user is an instruction to activate a function included in the basic function group.

2. The electronic sphygmomanometer according to claim 1, further comprising a display unit (4); wherein
the basic function group further includes a first displaying function of displaying the measurement result information stored in the first storage part on the display unit.

3. The electronic sphygmomanometer according to claim 2, wherein
each measurement result information further includes condition specifying data for specifying a measurement condition in blood pressure measurement in association with each blood pressure data; and
the information changing function includes at least one of an erasing function of erasing the measurement result information stored in the first storage part or a changing function of changing a value that becomes a reference of the measurement condition.

4. The electronic sphygmomanometer according to claim 3, wherein the setting portion sets the activation of the associated function group to enable when determined as started with the specific operation by the determining portion (2011), and sets the activation of the associated function group to disable when determined as started with an operation different from the specific operation by the determining portion.

5. The electronic sphygmomanometer according to claim 3, further comprising a timer (13) for measuring time; wherein
the condition specifying data includes timing data from the timer in the blood pressure measurement.

6. The electronic sphygmomanometer according to claim 3, further comprising a second storage part (122) for storing history information of the start-up with the specific operation; wherein
the associated function group further includes a second displaying function of displaying the history information stored in the second storage part on the display unit.

7. The electronic sphygmomanometer according to claim 6, further comprising a timer (13) for measuring date and time, wherein
the activation controlling part further includes a data storing portion (2013) for storing start-up date and time data in the second storage part as the history information based on an output from the timer when determined as started with the specific operation by the determining portion.

8. The electronic sphygmomanometer according to claim 6, wherein the activation controlling part further includes,
a counting portion (2013) for counting the number of start-ups with the specific operation when determined as started with the specific operation by the determining portion, and
a data storing portion (2013) for storing data of the counted number of start-ups in the second storage part as the history information.

9. The electronic sphygmomanometer according to claim 1, wherein the specific operation is an operation different from an explicit normal start-up operation.

10. The electronic sphygmomanometer according to claim 9, wherein
the first input unit includes a plurality of switches (21.0 to 21.6) that can be pushed by the user; and
the determining portion determines as started with the specific operation when two or more predetermined switches of the plurality of switches are simultaneously pushed by the user.

11. The electronic sphygmomanometer according to claim 9, further comprising
a second input unit arranged hidden from the outside; wherein the determining portion determines as started with the specific operation when the second input unit is operated.

## Patentansprüche

1. Elektronisches Sphygmomanometer, aufweisend:
eine Blutdruckmesseinheit (30), welche eingerichtet ist, Blutdruck zu messen;
einen ersten Speicherteil (121), welcher eingerichtet ist, Messergebnisinformation zu speichern, welche vorausgehend gemessene Daten des von der Blutdruckmesseinheit gemessen Blutdrucks enthält; und
eine erste Eingabeeinheit (21), welche eingerichtet ist, einem Benutzer ein Eingeben einer Anweisung zu ermöglichen, eine aus einer Mehrzahl von Funktionen zu aktivieren, welche in einer Basisfunktionsgruppe und einer angeschlossenen Funktionsgruppe des elektronischen Sphygmomanometers enthalten sind, wobei die Basisfunktionsgruppe eine Messfunktion der Blutdruckmesseinheit enthält und die angeschlossene Funktionsgruppe eine Informationsveränderungsfunktion in Bezug auf ein Löschen eines Messwerts oder ein Ändern eines Sollwerts enthält; und
einen Aktivierungssteuerungssteil (201), welcher eingerichtet ist, eine Steuerung auszuführen, eine der Mehrzahl von Funktionen basierend auf der Anweisung der ersten Eingabeeinheit zu aktivieren, **dadurch gekennzeichnet, dass**
der Aktivierungssteuerungsteil aufweist:
einen Feststellungsabschnitt (2011), weicher eingerichtet ist festzustellen, ob das elektronische Sphygmomanometer mit einer normalen oder einer besonderen Operation in Betrieb genommen wird,
einen Einstellungsabschnitt (2012), welcher eingerichtet ist, wenn vom Feststellungsabschnitt (2011) festgestellt wird, dass das elektronische Sphygmomanometer mit der besonderen Operation in Betrieb genommen wird, einen Entsperrmodus zu setzen, welcher die Aktivierung einer Funktion, welche in der angschlossenen Funktionsgruppe enthalten ist, freigibt, und welcher eingerichtet ist, wenn vom Feststellungsabschnitt (2011) festgestellt wird, dass das elektronische Sphygmomanometer mit der normalen Operation in Betrieb genommen wird, einen Sperrmodus zu setzen, welcher die Aktivierung der angeschlossenen, von der Basisfunktionsgruppe verschiedenen, Funktionsgruppe sperrt;
wobei der Aktivierungssteuerungsteil (201) ferner eingerichtet ist, die der Anweisung entsprechende Funktion unabhängig davon, ob der Sperrmodus oder der Entsperrmodus gesetzt ist, zu aktivieren, wenn die Anweisung des Benutzers eine Anweisung zur Aktivierung einer Funktion ist, welche in der Basisfunktionsgruppe enthalten ist.

2. Elektronisches Sphygmomanometer gemäß Anspruch 1, welches ferner eine Anzeigeeinheit (4) aufweist; wobei
die Basisfunktionsgruppe ferner eine erste Anzeigefunktion zum Anzeigen der Messergebnisinformation, welche im ersten Speicherteil gespeichert ist, auf der Anzeigeeinheit enthält.

3. Elektronisches Sphygmomanometer gemäß Anspruch 2, wobei
jede Messergebnisinformation ferner eine Bedingung angebende Daten zur Angabe einer Messbedingung bei der Blutdruckmessung in Verbindung mit den einzelnen Blutdruckdaten enthält; und
wobei die Informationsveränderungsfunktion wenigstens eine Löschfunktion zum Löschen der Messergebnisinformation, welche im ersten Speicherteil gespeichert ist, oder eine Änderungsfunktion zum Ändern eines Wertes, welcher zu einer Referenz der Messbedingung wird, enthält.

4. Elektronisches Sphygmomanometer gemäß Anspruch 3, wobei der Einstellungsabschnitt die Aktivierung der angeschlossenen Funktionsgruppe auf Freigegeben setzt, wenn eine Inbetriebnahme mit der besonderen Operation vom Feststellungsabschnitt (2011) festgestellt wird, und die Aktivierung der zugehörigen Funktionsgruppe auf gesperrt setzt, wenn eine Inbetriebnahme mit einer von der besonderen verschiedenen Operation vom Feststellungsabschnitt festgestellt wird.

5. Elektronisches Sphygmomanometer gemäß Anspruch 3, welches ferner einen Zeitgeber (13) zum Messen einer Zeit aufweist;
wobei die die Bedingung angebenden Daten Zeitdaten des Zeitgebers bei der Blutdruckmessung enthalten.

6. Elektronisches Sphygmomanometer gemäß Anspruch 3, welches ferner einen zweiten Speicherteil (122) zum Speichern von Verlaufsinformation des Inbetriebnahmevorgangs mit der besonderen Operation aufweist;
wobei die angeschlossene Funktionsgruppe ferner eine zweite Anzeigefunktion zum Anzeigen der im zweiten Speicherteil gespeicherten Verlaufsinformation auf der Anzeigeeinheit enthält.

7. Elektronisches Sphygmomanometer gemäß Anspruch 6, welches ferner einen Zeitgeber (13) zum Messen eines Datums und einer Zeit aufweist, wobei
der Aktivierungssteuerungsteil ferner einen Datenspeicherungsabschnitt (2013) zum Speichern von Inbetriebnahmedatumsdaten und Inbetriebnahmezeitdaten in dem zweiten Speicherteil als die Verlaufsinformation beruhend auf der Ausgabe des Zeitgebers, wenn vom Feststellungsabschnitt eine Inbetriebnahme mit der besonderen Operation festgestellt wird, aufweist.

8. Elektronisches Sphygmomanometer gemäß Anspruch 6, wobei der Aktivierungssteuerungsteil ferner aufweist:
einen Zählabschnitt (2013) zum Zählen der Anzahl von Inbetriebnahmevorgängen mit der besonderen Operation, wenn vom Feststellungsabschnitt eine Inbetriebnahme mit der besonderen Operation festgestellt wird, und
einen Datenspeicherungsabschnitt (2013) zur Speicherung von Daten der gezählten Anzahl von Inbetriebnahmen im zweiten Speicherteil als die Verlaufsinformationen.

9. Elektronisches Sphygmomanometer gemäß Anspruch 1, wobei die besondere Operation eine Operation unterschiedlich von einer ausdrücklich normalen Inbetriebnahmeoperation ist.

10. Elektronisches Sphygmomanometer gemäß Anspruch 9, wobei
die erste Eingabeeinheit eine Mehrzahl von Schaltern (21.0 bis 21.6) aufweist, welche vom Benutzer gedrückt werden können; und
der Feststellungsabschnitt eine Inbetriebnahme mit der besonderen Operation feststellt, wenn zwei oder mehr vorher festgelegte Schalter der Mehrzahl von Schaltern gleichzeitig von dem Benutzer gedrückt werden.

11. Elektronisches Sphygmomanometer gemäß Anspruch 9, ferner aufweisend:
eine zweite Eingabeeinheit, welche von außen nicht sichtbar angeordnet ist; wobei
der Feststellungsabschnitt eine Inbetriebnahme mit der besonderen Operation feststellt, wenn die zweite Eingabeeinheit betätigt wird.

## Revendications

1. Sphygmomanomètre électronique, comprenant :
une unité de mesure de pression artérielle (30) adaptée pour mesurer une pression artérielle ;
une première partie de stockage (121) adaptée pour stocker des informations de résultat de mesure comprenant des données mesurées antérieurement de la pression artérielle mesurée par l'unité de mesure de pression artérielle ; et
une première unité d'entrée (21) adaptée pour permettre à un utilisateur d'entrer une instruction pour activer une parmi une pluralité de fonctions incluses dans un groupe de fonctions de base et un groupe de fonctions associées du sphygmomanomètre électronique, le groupe de fonctions de base comprenant une fonction de mesure par l'unité de mesure de pression artérielle, et le groupe de fonctions associées comprenant une fonction de changement d'informations connexe à l'effacement d'une valeur de mesure ou au changement d'une valeur nominale ; et
une partie de commande d'activation (201) adaptée pour réaliser une commande pour activer une parmi la pluralité de fonctions selon l'instruction à partir de la première unité d'entrée ; **caractérisé en ce que**,
la partie de commande d'activation comprend :
une partie de détermination (2011) adaptée pour déterminer si le sphygmomanomètre électronique est démarré avec une opération normale ou avec une opération spécifique,
une partie de réglage (2012) adaptée pour régler, lorsqu'il est déterminé par la partie de détermination (2011) que le sphygmomanomètre électronique est démarrée avec l'opération spécifique, un mode de déverrouillage permettant l'activation d'une fonction incluse dans le groupe de fonctions associées et pour régler, lorsqu'il est déterminé par la partie de détermination (2011) que le sphygmomanomètre électronique est démarré avec l'opération normale, un mode de verrouillage désactivant l'activation du groupe de fonctions associées autre que le groupe de fonctions de base ;
la partie de commande d'activation (201) étant en outre adaptée pour activer la fonction correspondant à l'instruction, indépendamment du fait que le mode de verrouillage ou le mode de déverrouillage est réglé si l'instruction à partir de l'utilisateur est une instruction pour activer une fonction incluse dans le groupe de fonctions de base.

2. Sphygmomanomètre électronique selon la revendication 1, comprenant en outre une unité d'affichage (4) ; dans lequel
le groupe de fonctions de base comprend en outre une première fonction d'affichage de l'affichage des informations de résultat de mesure stockées dans la première partie de stockage sur l'unité d'affichage.

3. Sphygmomanomètre électronique selon la revendication 2, dans lequel
chaque information de résultat de mesure comprend en outre des données de spécification de condition pour spécifier une condition de mesure dans la mesure de pression artérielle en association avec chaque donnée de pression artérielle ; et
la fonction de changement d'informations comprend au moins une parmi une fonction d'effacement de l'effacement des informations de résultat de mesure stockées dans la première partie de stockage ou une fonction de changement du changement d'une valeur qui devient une référence de la condition de mesure.

4. Sphygmomanomètre électronique selon la revendication 3, dans lequel la partie de réglage permet l'activation du groupe de fonctions associées lorsqu'il est déterminé, par la partie de détermination (2011), que le démarrage est réalisé avec l'opération spécifique, et ne permet pas l'activation du groupe de fonctions associées lorsqu'il est déterminé, par la partie de détermination, que le démarrage est réalisé avec une opération différente de l'opération spécifique.

5. Sphygmomanomètre électronique selon la revendication 3, comprenant en outre un chronomètre (13) pour mesurer le temps ; dans lequel
les données de spécification de condition comprennent des données de chronométrage à partir du chronomètre dans la mesure de pression artérielle.

6. Sphygmomanomètre électronique selon la revendication 3, comprenant en outre une seconde partie de stockage (122) pour stocker des informations historiques du démarrage avec l'opération spécifique ; dans lequel
le groupe de fonctions associées comprend en outre une seconde fonction d'affichage pour afficher les informations historiques stockées dans la seconde partie de stockage sur l'unité d'affichage.

7. Sphygmomanomètre électronique selon la revendication 6, comprenant en outre un chronomètre (13) pour mesurer une date et une heure, dans lequel
la partie de commande d'activation comprend en outre une partie de stockage de données (2013) pour stocker des données de date et d'heure de démarrage dans la seconde partie de stockage en tant qu'informations historiques en fonction d'une sortie à partir du chronomètre lorsqu'il est déterminé, par la partie de détermination, que le démarrage est réalisé avec l'opération spécifique.

8. Sphygmomanomètre électronique selon la revendication 6, dans lequel la partie de commande d'activation comprend en outre :
une partie de comptage (2013) pour compter le nombre de démarrages avec l'opération spécifique lorsqu'il est déterminé, par la partie de détermination, que le démarrage est réalisé avec l'opération spécifique, et
une partie de stockage de données (2013) pour stocker des données du nombre compté de démarrages dans la seconde partie de stockage en tant qu'informations historiques.

9. Sphygmomanomètre électronique selon la revendication 1, dans lequel l'opération spécifique est une opération différente d'une opération de démarrage normal explicite.

10. Sphygmomanomètre électronique selon la revendication 9, dans lequel
la première unité d'entrée comprend une pluralité d'interrupteurs (21.0 à 21.6) sur lesquels l'utilisateur peut appuyer ; et
la partie de détermination détermine que le démarrage est réalisé avec l'opération spécifique lorsque l'utilisateur appuie simultanément sur deux, ou plus, interrupteurs prédéterminés parmi la pluralité d'interrupteurs.

11. Sphygmomanomètre électronique selon la revendication 9, comprenant en outre
une seconde unité d'entrée agencée de façon camouflée de l'extérieur ; dans lequel
la partie de détermination détermine que le démarrage est réalisé avec l'opération spécifique lorsque la seconde unité d'entrée est mise en fonctionnement.
